# EUROPEAN PATENT SPECIFICATION

(11) **EP 0 458 883 B1**
(45) Date of publication and mention of the grant of the patent: **13.11.1996**
(21) Application number: 90903691.5
(22) Date of filing: 12.02.1990
(51) Int. Cl.: A61B 5/00, A61B 5/0408, A61B 5/0416

(54) **WIRELESS ELECTROCARDIOGRAPHIC MONITORING SYSTEM**
DRAHTLOSES ELEKTROKARDIOGRAPHISCHES ÜBERWACHUNGSSYSTEM
SYSTEME DE CONTROLE ELECTROCARDIOGRAPHIQUE SANS FIL

(30) Priority: 15.02.1989 US 310660; 07.02.1990 US 473887
(43) Date of publication of application: 04.12.1991
(73) Proprietor: SEGALOWITZ, Jacob, Beverly Hills, CA 90212 (US)
(72) Inventor: SEGALOWITZ, Jacob, Beverly Hills, CA 90212 (US)
(74) Representative: Cross, Rupert Edward Blount
(86) International application number: US9000672
(87) International publication number: WO9009143

(56) References cited:
- EP-A- 0 212 278
- CH-A- 293 560
- US-A- 2 298 125
- US-A- 3 757 778
- US-A- 4 121 573
- US-A- 4 356 486
- US-A- 4 827 943
- ENCYCLOPEDIA OF MEDICAL DEVICES AND INSTRUMENTATION vol. 2, 1988, NEW YORK (US) pages 1002 - 1017; J. G. WEBSTER: 'ELECTROCARDIOGRAPHIC MONITORS'

## Description

This invention relates to medical instrumentation and, more particularly, to electrocardiographic and cardiocirculatory monitoring equipment.

### Prior Art

Every muscle can perform only one movement, the shortening of its fibers by contraction. This also applies to the heart muscle. Every action of a muscle has associated with it an electrical activity which changes in the course of the contraction. The electrical signal thus associated with the muscle action is transmitted through various tissues and ultimately reaches the surface of the body whereupon such electrical signals can be detected by electrodes applied to the skin. Thus, such signals that are being detected by the electrodes can be recorded with the aid of suitable electrocardiographic equipment or can be observed in or recorded with a monitor/recording unit. The record thus obtained is called an electrocardiogram or a rhythm-monitoring strip.

As early as 1855 action currents from the heart were recorded as measurements were being made of a beating frog heart. The first actual recording of a frog electrocardiogram was made by A.D. Waller in 1887. The first recording of a human heart electrical action signal (hereinafter "heart-signal") was made by A.D. Waller in 1889. Modern electrocardiography, however, started with Einthoven (credited with the bipolar lead triangle setting for recordings of standard limb leads I, II and III), who invented the string galvanometer and applied it to recording small voltages of short duration, which is the category into which heart-signals fall. His recording techniques have not been improved upon very much since they were first published many years ago. Here it should be noted that the term "lead" as used herein is being used in the medical sense and not the electronic sense (i.e., "lead" is a spatial position at which the heart-signal is viewed, not a wire).

After Einthoven's work, the entire field of research stagnated for nearly thirty years until the introduction by Wilson of upper and lower extremities' local leads and the zero electrode used in unipolar recordings.

The entire twelve-lead system is fed by unipolar and bipolar signals. Unipolar leads are divided into unipolar extremity or limb leads and unipolar precordial or chest leads.

The unipolar limb leads are:
aVR -- the unipolar right arm lead, (R designating the right arm);
aVL -- unipolar left arm lead, (L designating the left arm); and
aVF -- unipolar left leg lead;
in all of which the "a" stands for "augmented".

The unipolar chest leads are designated by the letter "V" followed by a subscript numeral which represents the exact location on the chest. In a standard electrocardiographic setting there are six precordial leads, V₁-V₆.

In standard bipolar leads, lead I is the potential difference between the arms, i.e., the left arm potential minus the right arm potential. Lead II is the potential difference between the left leg potential and the right arm potential. Lead III is the potential difference between the left leg and the left arm. If the leads are diagrammed on the body they inscribe, essentially, an equilateral triangle. The electrocardiograph generates the lead voltages from the potentials applied to it from the electrodes. The term "lead" as used in electrocardiography means view of the heart's electrical impulse. That view varies among leads.

The electrocardiograph is widely used by the medical profession. The standard electrocardiograph requires at least ten wires which are attached to the body of the patient at one end and to the electrocardiograph at the other end to detect heart-signals and transform them into a twelve-lead electrocardiogram evaluation. This involves attaching six electrodes to the chest or precordial area to obtain recordings of leads V₁-V₆ as well as attaching four electrodes to the arms and legs of the patient to obtain recordings of leads I, II, III, aVR, aVL and aVF. For heart rhythm monitoring, only three electrodes and three terminal wires are applied to the chest. After the ten electrodes are attached to the patient, ten specific wires must be connected between each specific electrocardiograph terminal and the related electrode of the predetermined position.

Many and frequent difficulties exist, and cumbersome operation of the conventional system arises due to the following factors:
1. Terminal wires from the electrocardiograph have to be connected to the distal electrodes in a predetermined order (defined limb and side to defined wire, as well as specific precordial points to defined precordial wires). In practice errors of connection between a specifically positioned electrode and the specifically associated wire from the electrocardiograph are relatively frequent.
2. The ten terminal wires often become intertangled with each other, and it takes precious time to untangle them.
3. When the electrocardiograph is to be operated by an Intensive Coronary Care Mobile Unit, which operates in relatively abnormal conditions, the speed of utilization of the system is often crucial since life-threatening situations are involved. In this environment the existing electrocardiographic system is somewhat impractical.
4. Since the terminal wires are reused, should they have any inherent manufacturing defects or if they develop such defects with use throughout their long extent (defects which are often difficult to detect), operation of the system (the electrocardiograph or monitor) is unsatisfactory.
5. During surgical procedures the patient is monitored for arrhythmias. Often the wires which extend beneath or beside the sterile field become disconnected from the electrodes positioned on the patient during the procedure, and it is difficult outside the sterile surgical field to reconnect the necessary wires. It is also time-consuming and interruptive of the surgical procedure, since monitoring of heart function is essential.
6. During hospitalization, rhythm monitored patients occasionally are permitted to ambulate within the department area. Often, the patient disconnects the wires at the bedside monitor, thus disconnecting the electrode signals from the monitor, in order to take a walk or to visit the rest room in the area. During this period no rhythm monitoring is possible.

CH-A-293 560 relates to electrocardiographic monitoring of a pilot in flight and describes an ECG monitoring method which varies from standard electrophysiologic basics by using an external common reference not on the person being monitored. A wireless transmission of the information obtained on the patient takes place. This document does not describe a method or apparatus for obtaining a standard twelve lead ECG from a patient.

US Patent No. 3,757,778 discloses an electrocardiograph lead distribution and contact testing apparatus which includes an electrocardiograph signal processor and recorder. The electrocardiograph input is electrically connected by a multiconductor patient cable to a remote distribution head. Lead wires are connected to electrodes which are adapted for connection with selected external portions of a patient for the sensing of body functions thereat and transmission of electrical impulses from the patient's circulatory system.

US Patent No. 4,121,573 discloses a system for monitoring a cardiac patient comprising a unit having a pair of spaced electrodes mounted on a resilient base which also carries a transmitter connected to the electrodes. A receiver-adapter for receiving the wireless transmitted signal from the transmitter is coupled to a conventional display device to provide a pictorial representation of the patient's heartbeat.

It is an object of this invention to overcome the problems previously experienced in connection with the application of electrocardiographs in the taking of electrocardiograms and in connection with the monitoring of patients.

It is a further object of this invention to provide an electrocardiographic and monitoring system in which the physical wires between the patient and the electrocardiograph or monitor are eliminated. It is a further object of this invention to provide an electrocardiographic and monitoring system in which a reduced standard number of wireless electrodes still provides a complete standard twelve-lead electrocardiogram.

According to the present invention there is provided a wireless electrocardiographic monitoring arrangement for monitoring a patient, comprising a right arm electrode having a conductive contact element for attachment to the patient in a position for receiving information with respect to the right arm of the patient, a left leg electrode having a conductive contact element for attachment to the patient for receiving information with respect to the left leg of the patient, a strip electrode for attachment to the chest of the patient extending from approximately the sternum of the patient toward the left arm of the patient, a set of six precordial conductive contact elements disposed on the strip electrode, a left arm conductive contact element disposed on the strip electrode in a location proximate to the left arm of the patient, a reference contact element carried by one of said electrodes; said right arm electrode, said left leg electrode and said strip electrode having seven conductive contact elements disposed thereon providing an electrode system, and wireless transmitting means carried by the electrode system for transmitting the information obtained by the electrode system from the patient to a location remote from the patient to provide a twelve lead electrocardiogram of the patient.

In accordance with the preferred embodiments of the present invention there is disclosed a two-section medical monitoring apparatus. One section includes the electrodes that are affixed to the patient for detection of and/or analysis of specific electrical signals, and the second section is the receiving equipment for analyzing the signals. The second section is used in connection with a monitor or ECG printer for displaying the signals detected by the electrodes without any wires extending between the first two sections. Each wireless electrode operates independently of all of the other electrodes and is self-contained and self-powered and individually radiates its measured signal to a corresponding individual receiver in the monitoring equipment section. To ensure that the receivers in the monitoring-receiving section operate on the proper signal from the electrodes, each of the electrodes transmits its signal with an encoded pattern that can be decoded only by its corresponding receiver in the monitoring unit. Additionally, in one embodiment of the present invention, one of the electrodes to be connected to the patient includes a receiver, and the monitoring unit contains a corresponding transmitter which is also encoded to prevent the receiver from operating on the wrong signal.

Embodiments of the present invention also present several different configurations, or groupings, of the ten electrodes needed for a "complete" standard twelve-lead electrocardiogram (ECG) monitoring. By grouping the electrodes, while maintaining their individual proper function, the number of appliances that have to be affixed to the patient is reduced, thus reducing the amount of time necessary to apply the electrodes and further minimizing the chance of error from the installation of the electrodes in the wrong locations on the patient.

The electrocardiac activity information detected and transmitted by the preferred embodiment conforms to all professional standards and levels of accuracy for vector progression, duration, intensity, and form characteristics specifically with respect to the following features:
1. Rhythm
2. Rate
3. P wave
4. P-R interval
5. QRS interval
6. QRS complex
7. ST segment
8. T wave
9. U wave
10. Q-T duration

Preferably the system will function within a nominal range of approximately 50 meters between the electrode system and the monitoring-receiving unit. This configuration is suitable for operation with either a single-channel or a multi-channel electrocardiograph, monitor, or Holter. Each of those units may be fixed or portable, battery or AC powered. The receiving-demodulating-decoding base unit of a preferred embodiment of the present invention can be connected to existing stand-alone electrocardiographs or, by reason of its miniature size, can be integrated into new generations of such machines. Interference between multiple systems operating in the same facility is prevented by choosing different center frequencies for each of the transmitters and corresponding receivers within the corresponding system and all other systems within 100 meters or more of each other.

The electrodes may be circular with concentric outer ring and center skin contacts, the outer ring being the reference potential contact to the body of the patient. Alternatively, an electrode with contacts that are spaced-apart from each other may be used, one being the signal or pick-up contact, and the other being the reference or zero potential contact.

While reference has been made herein to a radio frequency (RF) system of coupling between body electrodes and the monitoring-receiving base unit, it should be understood that with only minor changes in the circuitry and the proper operating environment, ultrasonic or light transmission techniques, as well as other technologies may be used.

In one embodiment, the unipolar, the left arm, right arm, and left leg limb signals are fed to a bridge or "Wilson" network in the base unit to derive a reference signal for application to the right leg reference or indifferent electrode. That reference signal is used to modulate a frequency modulation (FM) transmitter at the base unit. At the right leg electrode a battery operated receiver detects, decodes, and amplifies the indifferent or reference signal and applies it to the right leg through a two-contact electrode to complete the signal path in the system. Alternatively, a balanced, mixed combination of the left arm, right arm, and left leg signals is radiated from the base unit to the right leg RF receiver electrode which applies such combination signal to the right leg.

Referring to bipolar lead recordings, the electrodes function in the same wireless fashion with the switchable characteristic of each electrode permitting identification of the location of that electrode so that the proper combination of limb signals is utilized in the ECG to develop I, II, and III leads.

Similarly, the precordial electrodes V₁-V₆, according to a preferred embodiment of the invention, are coupled in wireless fashion to appropriate, respective channels in the base station for processing and use.

In the embodiments of the present invention the electrode on the right leg and the associated receiver and the corresponding transmitter in the base unit have been eliminated to further reduce the number of electrode assemblies that must be placed on the patient.

In further embodiments of the present invention, each transmitter includes:
noise coil means coupled to one of the conductive elements for detecting the background noise; and
differential input amplifier means having a non-inverting input terminal coupled to the other conductive element and an inverting terminal coupled to said noise coil means for subtracting the background noise signal from the signal present on the other conductive element.

Preferably the apparatus includes, in audition, an LED for heart-signal indication, and in one embodiment includes an LED and LED driver as part of each of said left arm, right arm, left leg and precordial transmitting means for indicating the presence of signals.

In one embodiment, the strip electrode includes adhesive means for affixing same to the chest of the patient and retaining it in the appropriate location. Preferably the strip electrode includes a body portion that is elastic to permit the stretching thereof to the length necessary to span the patient's chest from substantially the sternum to the left side.

In one embodiment, the centre frequency of each combination of corresponding transmitters and receivers is the same, and the centre frequency of each combination of corresponding transmitters and receivers is different from the centre frequency of each other combination of corresponding transmitters and receivers. Preferably each transmitter and receiver includes adjustment means for varying said centre frequency.

Alternatively, each combination of corresponding transmitters and receivers has the same digital encoding, and the digital encoding of each combination of corresponding transmitters and receivers is different from the digital encoding of each other combination of corresponding transmitters and receivers. Preferably each transmitter and receiver includes adjustment means for varying said digital encoding.

In one embodiment, the heart-signal transmitting means includes an FM transmitter.

This invention and its advances over the prior art can best be understood by reading the Specification which follows in conjunction with the drawing herein, in which:
Fig. 1 is a block diagram of a wireless electrocardiograph system;
Fig. 1A is a graphical representation of the distribution of additional electrodes in the system of Fig. 1;
Fig. 2 is a schematic diagram of a concentric electrode for use in one embodiment of the present invention;
Fig. 3 is a schematic diagram of a "Wilson" network or bridge for developing an indifferent signal;
Fig. 4 is a schematic diagram of an alternative circuit for developing a right leg signal;
Fig. 5 is an elevational view of the concentric electrode of Fig. 2;
Fig. 6 is an elevational view of an alternative form of electrode;
Fig. 7 is a block diagram of an electrode transmitter means with provision to switch the enabler code and the transmitter frequency;
Fig. 8 is a block diagram of a further wireless electrocardiograph system;
Fig. 9 is a block diagram of a wireless electrocardiograph system according to the preferred embodiment of the present invention;
Fig. 10A is a plan view of the electrical contact side of an electrode strip of one configuration for the six precordial electrodes for easy mounting on the chest of the patient;
Fig. 10B is a plan view of the electrical contact side of an electrode strip of a second configuration for the six precordial electrodes and the left side electrode for easy mounting on the chest of the patient according to the invention;
Fig. 11 is a cross-sectional side view of a snap mounting configuration for removably mounting a transmitter assembly to a disposable electrode;
Fig. 12A is a partial cut-away perspective view of a transmitter/electrode assembly of the present invention; and
Fig. 12B is a perspective view of a transmitter/electrode assembly of one embodiment of the present invention.

### Detailed Description of the Preferred Embodiments

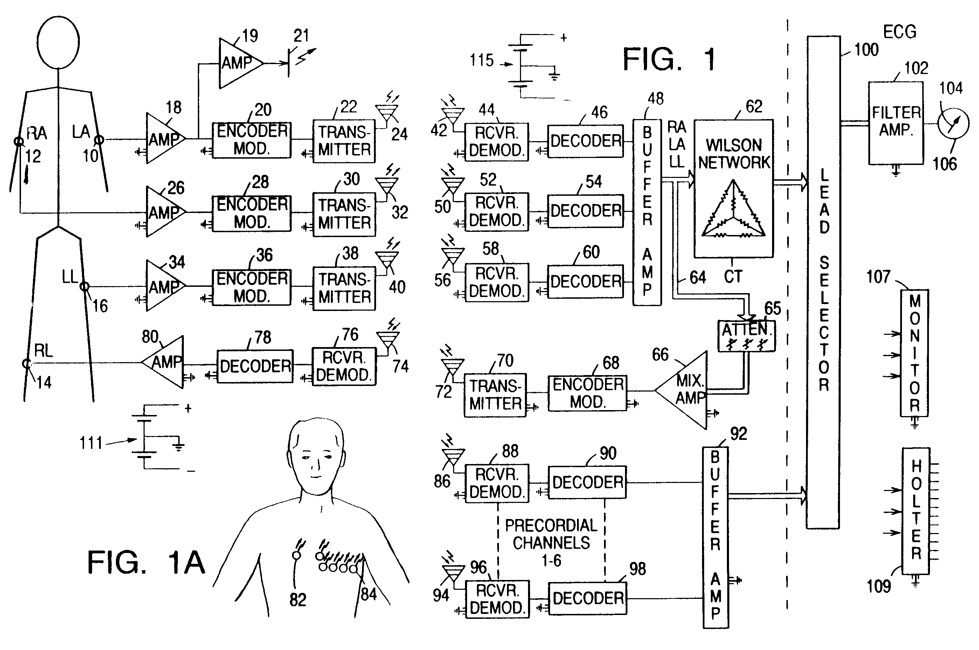

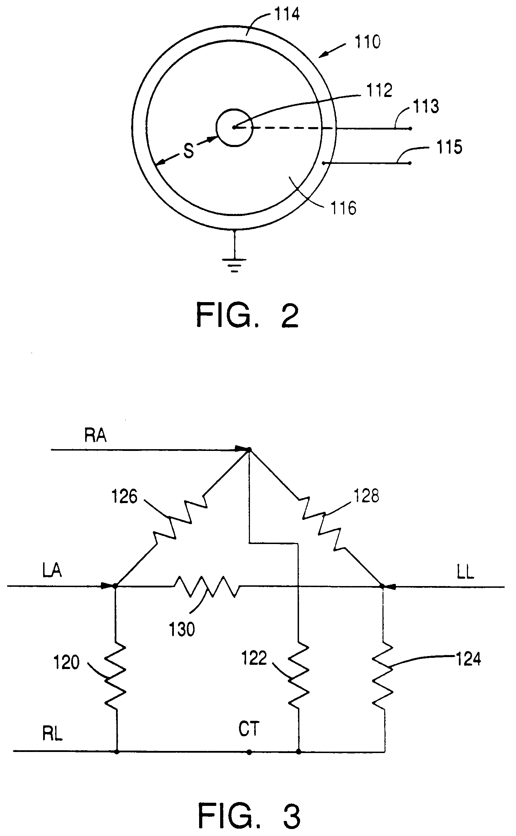

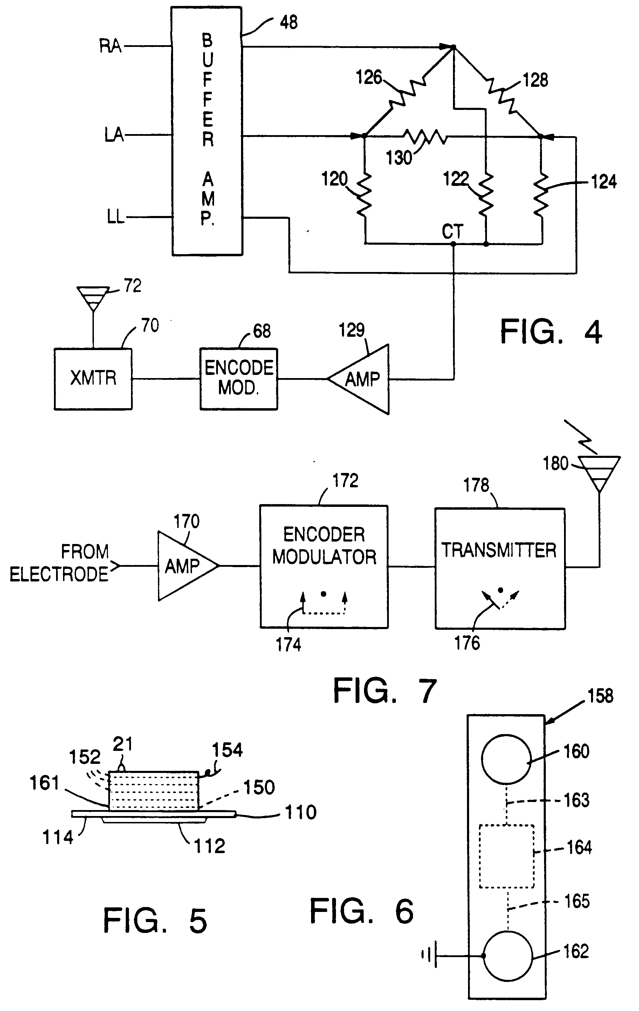

Fig. 1 shows a first embodiment of a wireless electrocardiographic monitoring apparatus. In Fig. 1, the human body is represented by a stick figure to show the location of limb electrodes of the system. Electrode 10 is connected to the left arm, electrode 12 is connected to the right arm, electrode 14 is connected to the right leg, and electrode 16 is connected to the left leg. Each of these electrodes requires two contactive surfaces which come into contact with the patient. They may be of either the concentric type shown in Fig. 2 or of the spaced-apart type of Fig. 6. In the Fig. 2 type the outer conductive strip is used to establish a localized zero or reference potential, and the center connector is the source of signal for transmission or the point of application of the signal in the receiving mode. The outer ring may be referred to as an indifferent electrode of a localized nature. In the Fig. 6 type, one contact is the signal contact, and the other contact is the reference or indifferent electrode.

The output signal from electrode 10 is fed to amplifier 18 which feeds encoder-modulator 20. The signal thus derived is used to modulate transmitter 22 which is connected to antenna 24 from which the modulated RF signal is radiated. Amplifier 18 may comprise a micro-chip-type RC 4560 which has a dual-stage operational amplifier. Encoder-modulator 20 may comprise a CM 8555 IPA, or the equivalent, in combination with a 40H393 chip. If digital encoding is utilized, a single transmitting frequency may be used for all transmitters. However, if analog modulation by the signal from electrode 10 is utilized, encoder-modulator 20 may act merely as a modulator, and each of the transmitters may be set at a different center frequency. Transmitter 22, and corresponding transmitters in other channels, may comprise a 930F5 micro-chip which includes a Colpitts oscillator. The audio frequency range which must be reproduced by the system is .05 Hz to 125 Hz. The FM swing of the carrier frequency is typically no higher than 40 percent of the carrier frequency.

As shown, the output signal of amplifier 18 may also be fed through on additional amplifier 19 to an LED 21 which will give a light pulse each time a heart-signal is received at electrode 10. This heart-signal indicator may be provided at each electrode if desired.

The heart-signal from electrode 12 is fed to an amplifier 26 which, again, is a high gain, low noise amplifier, and the output signal of amplifier 26 is fed to encoder-modulator 28 and then to transmitter 30. The output signal of transmitter 30 is fed to antenna 32 for radiation.

The heart-signal from left leg electrode 16 is fed to amplifier 34, and the output signal of amplifier 34 is fed to encoder-modulator 36 for modulating FM transmitter 38. The output signal of transmitter 38 is fed to antenna 40 for radiation.

The signal radiated by antenna 24 is intercepted by antenna 42 and fed to receiver-demodulator 44, the output signal of which is fed to decoder 46 and then to buffer amplifier 48.

The signal from antenna 32 is intercepted by antenna 50 and fed to receiver-demodulator 52, the output signal of which is fed to decoder 54 and then to buffer amplifier 48.

Similarly, the signal from antenna 40 is received by antenna 56, and that signal is demodulated in receiver-demodulator 58 which feeds its output signal to decoder 60 for application to buffer amplifier bank 48. It should be understood that in buffer amplifier bank 48 there is a series of amplifiers, one for each transmitting limb signal channel. The signals from buffer amplifier 48 are fed to what is known as a "Wilson" network. This is essentially a bridge, the make up of which can be seen in Fig. 3. The left arm, right arm, and left leg signals are coupled to the "Wilson" network to produce what is known as an indifferent or reference potential which appears at the central terminal (CT). In this embodiment the signal at CT is not used, but it is used in the embodiment of Fig. 4. Instead, here, the right arm, left arm, and left leg signals are fed to a balancing attenuator 65 (three independently variable impedance paths) and then to a mixer amplifier 66 where the mixed signal is amplified by an internal low noise, high gain amplifier. The output signal of mixer amplifier 66 is applied to encoder-modulator 68 to generate the signal to modulate transmitter 70 which is coupled to antenna 72 for radiation by antenna 72. The radiated reference signal is picked up by receiving antenna 74, such antenna being coupled to receiver-demodulator 76 which develops a signal in the audible or sub-audible frequency range. Such signal, if it is encoded, may then be decoded by decoder 78, and the resulting signal may be applied to amplifier 80 which is coupled to the active contact of electrode 14 carried by the right leg. This establishes the so-called zero potential on the indifferent or reference electrode 14. Such a zero signal is necessary for operation of a system involving the use of unipolar limb leads. The signals from the same electrodes are used to produce the bipolar limb leads previously described.

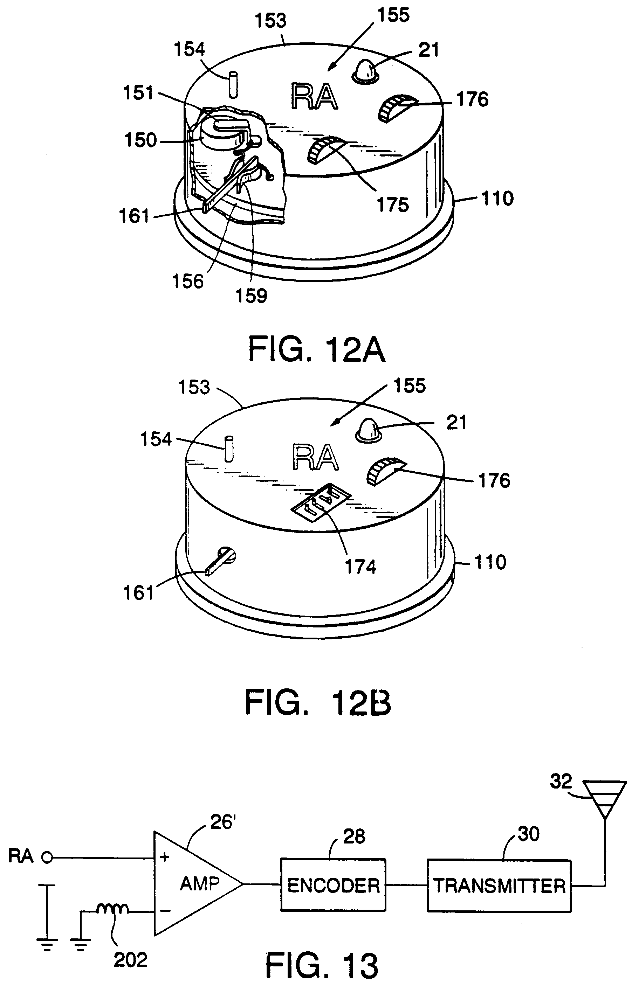

The transmitters in the system, if purely analog techniques are involved, may be set at center frequencies of, for example, 72.080 MHz and at multiples of 160 KHz around that center frequency. Since the transmitters operate simultaneously with 160 KHz separation between their center frequencies there is no problem with intermodulation at the respective receivers, each of which has a corresponding detector center frequency so that the proper received signal is acted upon. Operation at much higher frequencies, for example, in the 400 MHz band, results in a much shorter antenna requirement but increases power requirements, thus putting a heavier load on the very small battery which can be mounted with the micro-chips in connection with the electrodes utilized in the system, as shown in Figs. 5, 12A, and 12B. This analysis applies equally to electrodes used to detect signals for unipolar or bipolar leads. With a digitally encoded system, the center frequency may also be changed between systems to reduce the possibility of interference between systems operating in close proximity to each other.

As for the heart-signals at the precordial electrodes, such as electrode 82 and electrode 84, the transmitters are as shown in connection with the limb signal transmitters just described. Again, the frequencies are set differently, each from the other, but at higher frequencies; this is not a problem. Also, because the field strength of the signals from the various electrodes associated with transmitters is low, there is considerable freedom in choosing a frequency which is free of local interference. There are generally six precordial electrodes, and, therefore, in this system there are six precordial channels (for simplicity, only the first and sixth are shown in Fig. 1; however, each channel not shown is similar to those shown), each having the transmitting and receiving structures of corresponding elements in the limb signal channels. For example, the signal from the first precordial electrode 82 and its associated transmitter is received by antenna 86 and is fed to receiver-demodulator 88 where a signal in the audible or sub-audible frequency range is obtained and fed to decoder 90 for any decoding that is necessary to reproduce the heart-signal. The heart-signal thus derived is applied to buffer amplifier bank 92. Similarly, the heart-signal detected at the sixth precordial electrode 84 is transmitted by the associated transmitter and is received by antenna 94, following which it is detected and demodulated by receiver-demodulator 96, and, if necessary, it is then decoded by decoder 98 and fed to buffer amplifier 92. Buffer amplifier 92 is a bank of amplifiers, one for each of the six precordial signal channels. The limb signals and the precordial signals are fed, without loss of integrity, to lead selector 100 which is of the conventional type found in commercially available electrocardiographs and which permit selection of each of the channels individually. The output signals of lead selector 100 are fed to filter-amplifier 102, following which the signals are fed to the electrocardiographic analog or digital cardiographic display. The analog recording pens are represented by needle 104. The galvanometric mechanism is represented by element 106. Alternately, selected ones of the signals shown entering lead selector 100 may be fed directly to a monitor 107 or to a Holter system 109 for developing a twelve-lead electrocardiogram from the signals sensed by the electrodes placed only on the chest area.

Turning to Fig. 2, there is shown one electrode patch configuration for use with one embodiment of the present invention. Electrode patch 110 includes signal contact element 112 that is electrically conductive in nature and has a conductor 113 associated therewith for coupling, for example, to the micro-chip amplifier, the encoder-modulator, the transmitter element of Fig. 1, or to external equipment. Contact 112 may be made of aluminum, for example. Contact 114 is the indifferent or reference contact with the spacing "S" between contact 114 and contact 112 being typically 2-4 cm. Of course, concentric ring contact 114 is electrically conductive in nature. In some cases it may not be a closed circle but may merely be an arc of a circle. Contacts 112 and 114 are carried on an electrically non-conductive plastic film body 116, for example. If contacts 114 and 112 are too widely separated, the accuracy of the graphic reproduction of the heart-signal will be diminished.

In Fig. 3 the so-called "Wilson" network is shown. The purpose of this network is to establish a zero area of the field from the heart dipole which is creating the field being studied. As can be seen from Fig. 3, the right arm, left arm, and left leg potentials are combined through three equal resistors 120, 122, and 124 to establish a zero or reference point which is generally referred to as the central terminal. The size of each of resistors 120, 122, and 124 is in excess of 5,000 ohms with the general range being 5,000 - 15,000 ohms. The CT potential is not actually zero. Theoretically, the potential of the CT is the mid-dipole potential of the heart-signal generator if the field is homogenous and if the dipole generating the signal lies exactly in the center of an equilateral triangle, the angles of which are formed by the three electrode points LA, RA and LL. Resistors 126, 128 and 130 have the same resistance and form an electrical equilateral triangle simulating the Einthoven triangle of the electrocardiographic art.

According to the embodiment of Fig. 4, the CT potential, after amplification by amplifier 129, is transmitted by transmitter 70 through antenna 72 to receiver-demodulator 76 and its associated components for application to the right leg electrode 14, shown in Fig. 1.

Unipolar leads are presently the only ones used in the precordial positions, such as in Fig. 1A. It was formerly believed that a limb could serve as the indifferent or reference connection because it was relatively so distant from the precordial electrode. It soon became apparent, however, that this was not the case, that the arm or leg was not truly indifferent, and that it altered the results in varying degrees depending upon which limb was connected to the negative terminal of the electrocardiograph. Thus, there was a need to establish a central terminal, as described hereinbefore.

Associated with each of the limb and precordial electrodes is a power supply 111 (conventionally a battery) which provides to the micro-chips operating voltage of the necessary polarity and magnitude. At the base station side, integrated circuit (IC) operating voltage of the necessary polarity and magnitude is provided by power supply 115 which may be battery or AC based.

From Figs. 5, 12A, and 12B, it is apparent that each electrode patch 110 carries its own power supply 150 as well as the necessary micro-chips 152, which are powered by power Supply 150, and an antenna 154. An LED 21 may also be provided which lights with each heart beat. Tab 161 is an insulating member which, when pulled, connects power supply 150 to the micro-chips and activates the associated electrode assembly. This is true for the receiving electrode 14 on the right leg as well as for the various transmitting electrodes.

Figs. 12A and 12B are perspective views of a typical electrode assembly of one embodiment of the present invention. They each show an electrode assembly housing 153 mounted on an electrode 110. On top of housing 153 there is shown an electrode position indicator 155; in these views the letters "RA" designate that this electrode assembly is for use on the right arm of the patient. Other means for identifying the electrode assemblies can be used, such as color coding. No matter what form of coding is used, each of those codes needs to be designed to minimize the possibility of error caused by installation of the electrodes in the wrong location on the patient.

Also shown in these figures is a frequency change switch 176 which could be a detented wheel type switch so that each frequency position is well defined and the operator will easily know which setting the frequency is in. Similarly, there is an encoding selector switch in either a detented wheel switch configuration shown as switch 175, or a DIP switch 174.

Fig. 12A is also partially cut away to show the printed circuit board 156 that is internal to the assembly with a battery 150 mounted to board 156 and making contact thereto via battery clip 151. Serially connected to battery clip 151 is a pair of electrically conductive spring fingers 159 which are mounted adjacent each other. Between fingers 159 is tab 161 which prevents contact of fingers 159 with each other. The combination of tab 161 and fingers 159 therefore act as an on/off switch which prevents battery 150 from discharging prior to use and allows the operator to very positively activate the electrode assembly by completely removing tab 161. If such an assembly is to be reused, tab 161 should be reinserted between fingers 159 to deactivate the circuitry.

Fig. 6 shows an alternative form of electrode structure 158 which is particularly useful for precordial application. The necessary separation of signal electrode 160 and zero reference electrode 162 is achieved in the limited space available on the chest. This strip electrode structure carries a micro-chip amplifier, an encoder-modulator, and a transmitter module 164 (with battery) and connectors 163, 165 for signal input.

Each electrode may have a multiple-position switch which changes the frequency or digital encoding of the signal from the electrode so that it matches the parameter for the site at which it is to be used on the patient in order to limit the number of electrodes with different operating parameters which must be kept in inventory and employed in setting up the system for a given patient.

The electrodes may be color coded or labeled to indicate where they should be placed on the body. They may also include frequency or code switches to permit one electrode type to be usable in various body locations.

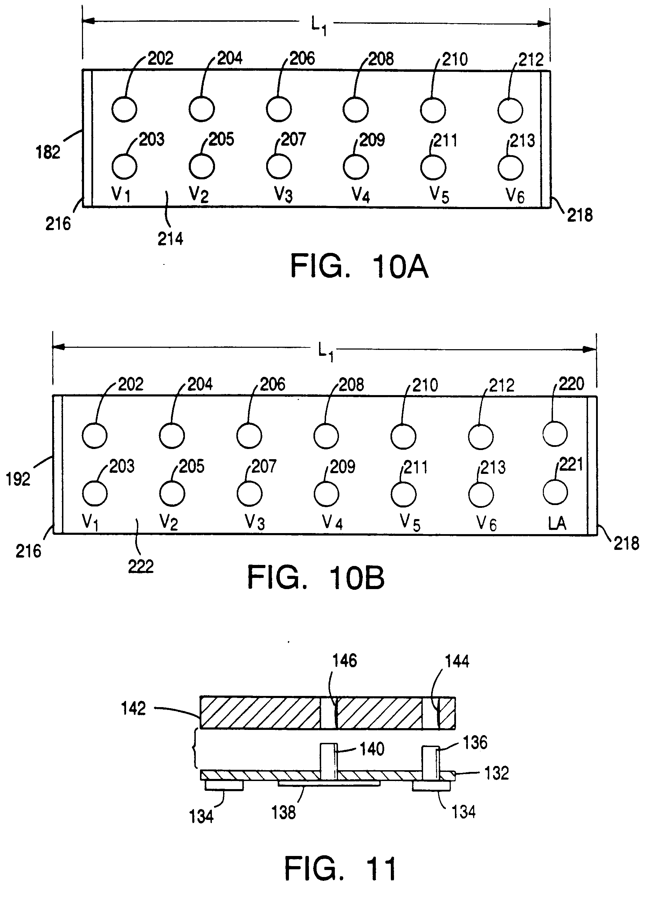

Fig. 11 illustrates another form of electrode/electronics assembly that may be of interest in some applications. This assembly consists of two portions, an electrode assembly 132 that may be disposable, and an electronics assembly 142 that may be reusable. Electrode assembly 132 includes reference contact 134 and its associated connection position post that extends through the insulative body of the electrode to the side opposite the contact, and signal contact 138 and its connection post 140 that also extends through the body of the electrode. Electronics assembly 142 is sized and configured to mount onto electrode assembly 132 and to mate with connection posts 136 and 140 which are disposed to be received by contacts 144 and 146, respectively. Each of the contacts 144 and 146 are spring loaded to make electrical contact with and to physically capture connection posts 136 and 140.

Fig. 7 illustrates in block form this capability. In Fig. 7, heart-signals from any of the ten electrodes are coupled to amplifier 170, as shown in Fig. 1. The output of amplifier 170 is fed to encoder-modulator 172 which includes a quad NAND gate section as is found, for example, in a type TSC-323 integrated circuit. DIP switch 174 permits selection of a three-digit code if digital encoding is used. On the other hand, if analog encoding is to be used, a frequency-change switch 176 is provided on transmitter 178. The oscillators described in connection with Fig. 1 (which are used here) are tunable by changing the applied voltage. Such voltage change is accomplished by using switch 176 to vary the voltage applied to the control line of a voltage controlled oscillator (VCO) that is a portion of the transmitter. Thus, by either method, the number of different types of electrodes that must be inventoried can be reduced. The base station decoders or receiver-demodulator may be fixed, and the electrode encoding means may be adjusted to correspond to the code or frequency of a target signal channel at the base station. A similarly equipped receiver-demodulator would also have to be provided to make similar adjustments either to the decoder or to the center frequency of the receiver.

The appropriately encoded signal, or the signal at the desired frequency, is fed to antenna 180.

This system is adapted to work with Holter systems in which twelve-lead electrocardiograms are derived from three-electrode information. This fact is illustrated by element 109 in Fig. 1.

The unipolar leads with respect to the right arm, left arm and left leg of the patient are traditionally measured with respect to the right leg as the reference point. With a system wherein each signal is individually transmitted to a base station, it is undesirable to run a wire from the right arm, left arm and left leg electrodes to the electrode attached to the right leg of the patient as a reference point. In the embodiment disclosed in Fig. 1, the reference point at the right leg is created from the signals at the other extremities of the patient in the base station and then transmitted to the right leg electrode, as discussed above. This is the ten-electrode assembly approach. The right leg has historically been used as the reference point substantially because the earlier instrumentation lacked the sensitivity and the noise rejection capabilities of today's electronic devices.

After additional experimentation it has been determined that a reference point that is closer to the signal electrode than the right leg can be used, and that each of the three signals necessary to determine the bipolar leads I, II and III, as well as the unipolar leads aVR, aVL and aVF, can be measured with respect to different reference points without reconstructing a common reference point signal. Today's electronics requires that each of these reference points be at least 2-4 cm. from the corresponding signal contact. An electrode such as that shown is either of Figs. 2 or 6 could be used for this purpose. The controlling factor is therefore the ability of the electronics to extract the needed signals from the background noise. This is the nine-electrode assembly approach.

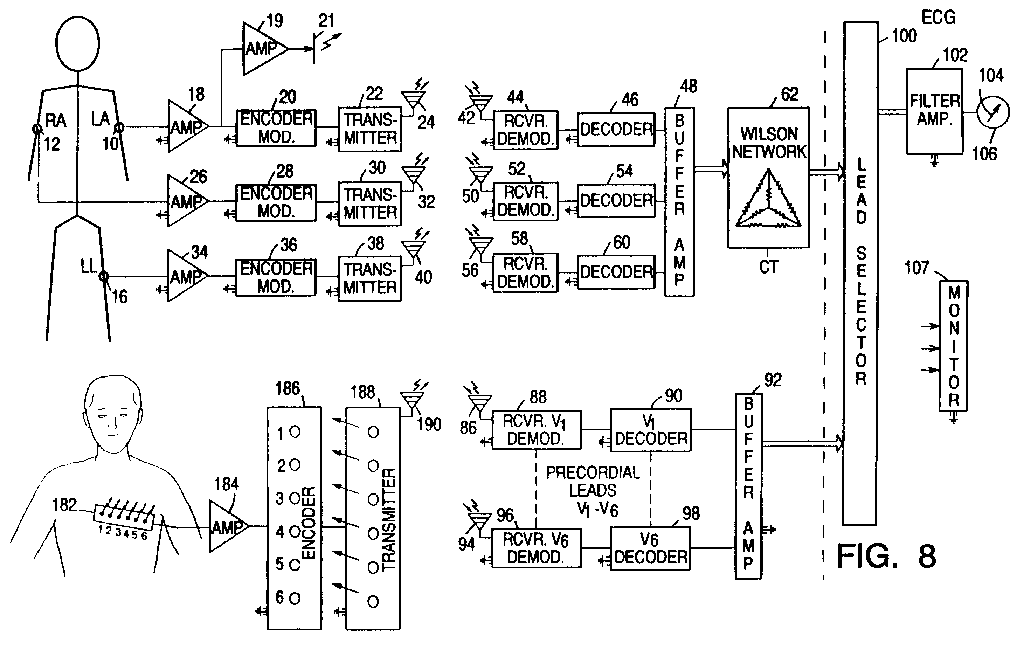

Therefore, a embodiment is shown in Fig. 8 which does not have the receiving and transmitting paths associated with the electrode on the right leg of the patient, as shown in Fig. 1.

In a continuing effort to make the wireless ECG system even more efficient and practical, a precordial electrode strip 182 has been designed. (See Figs. 8 and 10A.) Strip 182 includes a pair of contacts 202-213 (signal contacts are even numbered, and reference contacts are odd numbered) for each precordial signal measurement. Each of the contacts 202-213 is mounted on one side of strip 182 in a spaced-apart relationship to the other contact, and each of the contacts 202-213 has a connecting post, or the like, that extends through strip 182 to the opposite side. Precordial transmitter assemblies are mounted to the other side of strip 182 and electrically connected to the appropriate connecting post. The precordial electrodes are to be located on the body of the patient, evenly spaced, with the first being approximately 1 cm. to the left of the patient's sternum, and the sixth approaching the patient's side. To accomplish this, various lengths of precordial electrode strips 182 could be manufactured. Alternatively, the base material of strip 182 could be elastic, similar to an elastic bandage. To hold strip 182 to the chest of the Patient, adhesive areas 216 and 218 can be provided at either end of strip 182. Alternately, the entire contact side 214 of strip 182 can be coated with an adhesive other than on the faces of the twelve contacts. This is the four-electrode assembly approach, including individual right arm-, left arm-, and left leg-wireless electrode assemblies, and electrodes for V₁ through V₆ mounted on strip 182 to form an additional assembly.

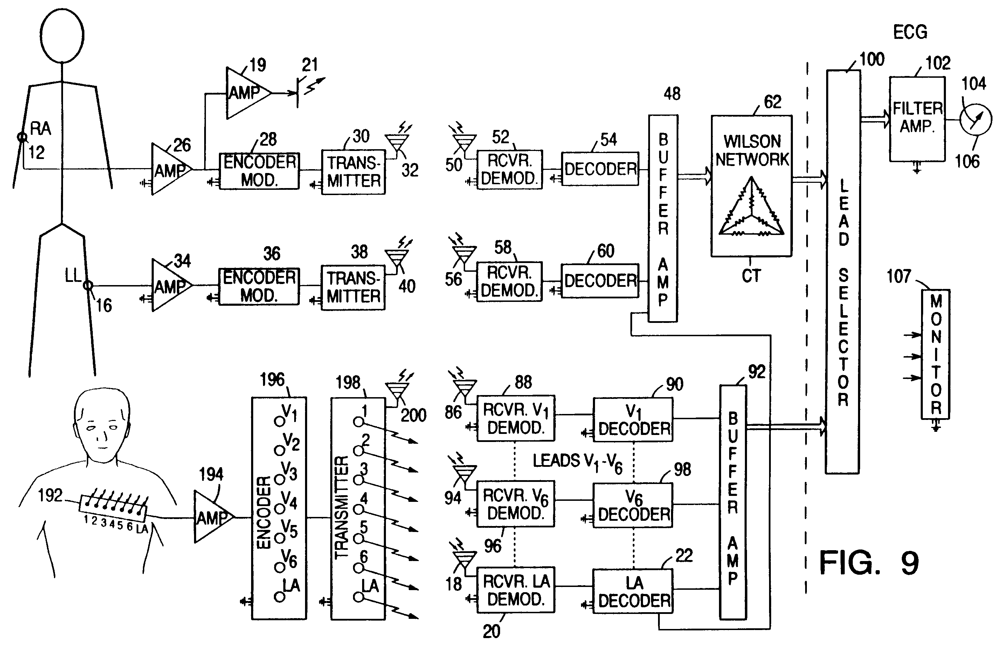

Additional experimentation has shown that the left arm electrode can be moved to the left side of the patient with no loss in accuracy of the resulting ECG data. As a result of this experimentation the preferred embodiment of the present invention foresees the lengthening of the precordial electrode chest strip of Fig. 10A to include a pair of contacts 220 and 221 to monitor the left arm signal at the left side of the patient. The extended strip 192 is shown in Fig. 10B, and tile corresponding electronics is shown in Fig. 9. This is the three-electrode assembly approach.

In either of the embodiments of Figs. 8 and 9 the transmitting assemblies attached to strip 182 or strip 192 may be individual units that are attached to the appropriate connection posts of the various electrodes, or they may be individual transmitters which are all contained within a common housing.

Reference to only a wireless ECG has been made up to this point in this Specification. However, the wireless transmission of signals from a patient, regardless of the function being monitored, can be transmitted and received remotely in the same way. Thus, a truly wireless monitoring system that includes pulse, temperature, etc. can be easily accomplished with the transmitting and receiving devices of the various embodiments of the present invention.

To further improve the possibility of the electrode assemblies to separate the desired signal from the background noise, amplifiers such as 26 and 34 can be differential input amplifiers as shown in Fig. 13. Differential input amplifier 26' includes two signal input terminals -- an inverting terminal and a non-inverting terminal. The signal from the right arm electrode is connected to the non-inverting terminal, and a noise pick-up coil 202 is connected to the inverting terminal. The purpose of noise pick-up coil 202 is to detect the background noise and to apply that to amplifier 26'. The theory is to subtract the background noise from the signal that is included with the signal from the right arm electrode. This is accomplished by the difference in sign of the two input terminals of amplifier 26' since the output signal from amplifier 26' is the arithmetic difference between the two input signals that have been amplified by a selected factor. Thus, including this type of amplifier in each of the transmitter assemblies will largely reduce the signal noise and permit the use of less sensitive components or will allow the use of electrode patches that have a smaller spacing between their signal and reference contacts.

## Claims

1. A wireless electrocardiographic monitoring arrangement for monitoring a patient, comprising a right arm electrode (12,110) having a conductive contact element (112) for attachment to the patient in a position for receiving information with respect to the right arm of the patient, a left leg electrode (16,110) having a conductive contact element (112) for attachment to the patient for receiving information with respect to the left leg of the patient, a strip electrode (192) for attachment to the chest of the patient extending from approximately the sternum of the patient toward the left arm of the patient, a set of six precordial conductive contact elements (203,205,207,209,211,213) disposed on the strip electrode (192), a left arm conductive contact element (221) disposed on the strip electrode (192) in a location proximate to the left arm of the patient, a reference contact element (114,202) carried by one of said electrodes; said right arm electrode (12, 110), said left leg electrode (16, 110) and said strip electrode (192) having seven conductive contact elements (203,205,207,209,211,213,221) disposed thereon providing an electrode system; and wireless transmitting means (152,198) carried by the electrode system for transmitting the information obtained by the electrode system from the patient to a location remote from the patient to provide a twelve lead electrocardiogram of the patient.

2. An arrangement as claimed in Claim 1, wherein said transmitting means (152,198) includes a plurality of separate spaced apart transmitters (30,38 198).

3. An arrangement as claimed in Claim 1 or 2, including receiving means (20,44,52,58,88,96) remote from the transmitting means (152,198) for receiving the transmitted information and for producing a twelve lead electrocardiogram therefrom.

4. An arrangement as claimed in any preceding claim, wherein said arm electrode includes a layer of insulating material (116) having first and second sides, a conductive contact element (112) carried by said layer of insulating material (116) and disposed on said first side of said layer of insulating material (116), a battery (150) carried on said second side of said layer of insulating material (116), microchip amplifier (152) and said transmitting means (154) carried on said second side of said layer of insulating material (116) and being coupled to said battery (150) and to said conductive contact element (112) for receiving heart signals from the heart of the patient and for transmitting wireless signals in accordance with heart signals received.

5. An arrangement as claimed in Claim 4, including an additional conductive contact element (114) carried by said layer of insulating material (116) and disposed on said first side of said layer of insulating material (116), said microchip amplifier and transmitting means (152,154) being coupled to said additional conductive contact element (114).

## Patentansprüche

1. Drahtlose elektrokardiographische Überwachungsanordnung zur Überwachung eines Patienten, mit einer Elektrode (12, 110) für den rechten Arm, die ein leitfähiges Kontaktelement (112) zur Befestigung am Patienten in einer Position zum Empfang auf den rechten Arm des Patienten bezogener Information aufweist, und einer Elektrode (16, 110) für das linke Bein, die ein leitfähiges Kontaktelement (112) zur Befestigung am Patienten in einer Position zum Empfang auf das linke Bein des Patienten bezogener Information aufweist, einer sich ungefähr vom Sternum des Patienten bis zum linken Arm des Patienten erstreckenden Streifenelektrode (192) zur Befestigung auf der Brust des Patienten, einer Anordnung von sechs auf der Steifenelektrode (192) angebrachten präkordialen, leitfähigen Kontaktelementen (203, 205, 207, 209, 211, 213), einem an einem dem linken Arm nahe gelegenen Ort des Patienten auf der Steifenelektrode (192) angebrachten leitfähigen Kontaktelement (221) für den linken Arm, einem auf einer der Elektroden angebrachten Referenz-Kontaktelement (114, 202), wobei die Elektrode (12, 110) für den rechten Arm, die Elektrode (16, 110) für das linke Bein und die Steifenelektrode (192) sieben darauf angeordnete leitfähige Kontaktelemente (203, 205, 207, 209, 211, 213, 221) aufweisen und so ein Elektrodensystem bilden; und einer auf dem Elektrodensystem angebrachten drahtlosen Sendeeinrichtung (152, 198) zum Senden der vom Elektrodensystem erfaßten Information vom Patienten an einen vom Patienten entfernten Ort zur Erstellung eines auf zwölf Leitungen basierenden Elektrokardiogramms des Patienten.

2. Anordnung nach Anspruch 1, bei der die Sendeeinrichtung (152, 198) mehrere voneinander entfernte Sender (30, 38, 198) aufweist.

3. Anordnung nach Anspruch 1 oder 2, mit von der Sendeeinrichtung (152, 198) entfernten Empfangseinrichtungen (20, 44, 52, 58, 88, 96) zum Empfangen der gesendeten Information und zum daraus Erzeugen eines auf zwölf Leitungen basierenden Elektrokardiogramms.

4. Anordnung nach einem der vorhergehenden Ansprüche, bei der die Armelektrode eine Schicht Isoliermaterial (116) mit einer ersten und einer zweiten Seite aufweist, einem leitfähigen Kontaktelement (112), das auf der Isoliermaterialschicht (116) angebracht und auf der ersten Seite der Isoliermaterialschcht (116) angeordnet ist, einer Batterie (150), die auf der zweiten Seite der Isoliermaterialschicht (116) angeordnet ist, wobei ein Mikrochipverstärker (152) und die Sendeeinrichtung (154) auf der zweiten Seite der Isoliermaterialschicht (116) angeordnet und an die Batterie (150) und das leitfähige Kontaktelement (112) zum Empfangen von Herzsignalen aus dem Herz des Patienten und zum Senden den empfangenen Herzsignalen entsprechender drahtloser Signale angeschlossen sind.

5. Anordnung nach Anspruch 4, mit einem zusätzlichen leitfähigen Kontaktelement (114), das auf der Isoliermaterialschicht (116) angebracht und auf der ersten Seite der Isoliermaterialschicht (116) angeordnet ist, wobei der Mikrochipverstärker und die Sendeeinrichtung (152, 154) an das zusätzliche leitfähige Kontaktelement (114) angeschlossen ist.

## Revendications

1. Appareil de surveillance électrocardiographique sans fil pour la surveillance d'un patient, comportant une électrode (12, 110) pour le bras droit ayant un élément de contact conducteur (112) destiné à être fixé au patient dans une position pour recevoir une information concernant le bras droit du patient, une électrode (16, 110) pour la jambe gauche ayant un élément de contact conducteur (112) destiné à être fixé au patient pour recevoir une information concernant la jambe gauche du patient, une électrode à barde (192) destinée à être fixée à la poitrine du patient, s'étendant d'un point proche du sternum du patient vers le bras gauche du patient, un jeu de six éléments de contact conducteurs précordiaux (203, 205, 207, 209, 211, 213) disposés sur l'électrode à bande (192), un élément de contact conducteur (221) de bras gauche disposé sur l'électrode à bande (192) en un emplacement proche du bras gauche du patient, un élément de contact et de référence (114, 202) porté par l'une desdites électrodes ; sept éléments de contact conducteurs (203, 205, 207, 209, 211, 213, 221) étant disposés sur ladite électrode (12, 110) du bras droit, ladite électrode (16 ,110) de la jambe gauche et ladite électrode (192) à bande et constituant un système d'électrodes ; et dos moyens (152, 198) d'émission sans fil portés par le système d'électrodes pour émettre l'information obtenue par le système d'électrodes depuis le patient jusqu'a un emplacement éloigné du patient afin de produire un électrocardiogramme à douze dérivations du patient.

2. Appareil selon la revendication 1, dans lequel lesdits moyens d'émission (152, 198) comprennent plusieurs émetteurs espacés et séparés (30, 38, 198).

3. Appareil selon la revendication 1 ou 2, comprenant des moyens de réception (20, 44 ,52, 58, 88, 96) éloignés des moyens d'émission (152, 198) pour recevoir les informations émises et pour produire, à partir d'elles, un électrocardiogramme à douze dérivations.

4. Appareil selon l'une quelconque des revendications précédentes, dans lequel ladite électrode de bras comprend une couche de matière isolante (116) ayant des premier et second côtés, un élément de contact conducteur (112) porté par ladite couche de matière isolante (116) et disposé sur ledit premier côté de ladite couche de matière isolante (116), une pile (150) portée sur ledit second côté de ladite couche de matière isolante (116), un amplificateur (152) à microplaquette et lesdits moyens d'émission (154) portés sur ledit second côté de ladite couche de matière isolante (116) et connectés à ladite pile (150) et audit élément de contact conducteur (112) pour recevoir des signaux promenant du coeur du patient et pour transmettre sans fil des signaux conformes aux signaux du coeur reçus.

5. Appareil selon la revendication 4, comprenant un élément de contact conducteur additionnel (114) porté par ladite couche de matière isolante (116) et disposé sur ledit premier côté de ladite couche de matière isolante (116), ledit amplificateur à microplaquette et lesdits moyens d'émission (152, 154) étant connectés audit élément de contact conducteur additionnel (114).
